# EUROPÄISCHE PATENTSCHRIFT

(11) **EP 1 023 609 B1**
(45) Veröffentlichungstag und Bekanntmachung des Hinweises auf die Patenterteilung: **20.06.2001**
(21) Anmeldenummer: 98956813.4
(22) Anmeldetag: 13.10.1998
(51) Int. Cl.: G01R 33/28, A61B 5/055

(54) **STENT UND MR-BILDGEBUNGSVERFAHREN ZUR DARSTELLUNG UND POSITIONSBESTIMMUNG EINES STENTS**
STENT AND MR IMAGING METHOD FOR REPRESENTING AND DETERMINING THE POSITION OF A STENT
EXTENSEUR ET PROCEDE D'IMAGERIE PAR RESONANCE MAGNETIQUE POUR REPRESENTER ET DETERMINER LA POSITION D'UN EXTENSEUR

(30) Priorität: 13.10.1997 DE 19746735
(43) Veröffentlichungstag der Anmeldung: 02.08.2000
(73) Patentinhaber: Simag GmbH, 12247 Berlin (DE)
(72) Erfinder: Melzer, Andreas, 45478 Mülheim/Ruhr (DE); Busch, Martin, Dr., 58455 Witten (DE)
(74) Vertreter: Müller, Wolfram Hubertus, Dipl.-Phys.
(86) Internationale Anmeldenummer: DE9803045
(87) Internationale Veröffentlichungsnummer: WO9919738

(56) Entgegenhaltungen:
- EP-A- 0 768 539
- EP-A- 0 775 500
- WO-A-96/38083
- US-A- 5 170 789

## Beschreibung

Die Erfindung betrifft ein MR (Magnetresonanz)-Bildgebungsverfahren zur Darstellung und Positionsbestimmung eines Stents nach dem Oberbegriff des Anspruchs 1 und einen Stent nach dem Oberbegriff des Anspruchs 8.

### Hintergrund der Erfindung

MR-Bildgebungsverfahren sind seit längerem bekannt. Sie beruhen auf der Resonanz-Wechselwirkung zwischen einem hochfrequenten elektromagnetischen Wechselfeld und bestimmten Atomkernen eines zu untersuchenden Objektes, insbesondere eines menschlichen oder tierischen Körpers, das in einem starken äußeren Magnetfeld angeordnet ist. Die Atomkerne präzedieren im Magnetfeld (B₀) mit der sogenannten Lamorfrequenz, die proportional zur Stärke des Magnetfeldes ist. Bei Einstrahlen eines elektromagnetischen Wechselfelds, dessen magnetische Wechselkomponente (B₁) senkrecht zur Richtung des starken Magnetfelds (B₀) ist, werden die Spins der Atomkerne zum Umklappen gebracht und können damit zusammenhängende Relaxationszeiten gemessen werden.

Für die Beschreibung in einem wissenschaftlichen Modell wird die Magnetisierung der einzelnen Spins zu einer Gesamtmagnetisierung zusammengefaßt. Diese Gesamtmagnetisierung ist in ihrer Gleichgewichtslage parallel zum äußeren Magnetfeld und wird Gleichgewichtsmagnetisierung genannt. Durch einen mit der Lamorfrequenz (Resonanzfrequenz) eingestrahlten HF-Impuls kann die Magnetisierung um einen Winkel α in Bezug auf die Magnetfeldrichtung ausgelenkt werden. Der Winkel α ist proportional zur Dauer des eingestrahlten HF-Impulses und zur Magnetfeldstärke (B₁) des HF-Impulses. Nach einer Anregung um den Winkel α präzediert die Gesamtmagnetisierung um die Richtung des Magnetfeldes. Die präzedierende Magnetisierung kann mit einer Spule, die senkrecht zur Richtung des Magnetfeldes orientiert ist, als ein Spannungssignal aufgezeichnet werden. Die Stärke des Spannungssignals ist proportional zu sin( α), proportional zur Dichte der Spins im signalgebenden Volumen und umgekehrt proportional zur Temperatur.

Die maximale Signalantwort eines gegebenen Volumens wird daher nach einer 90° Anregung erreicht. Die aufgezeichnete Signalamplitude nimmt exponentiell mit der Relaxationszeit T₂^{*} ab, da die einzelnen Spins auf Grund von fluktuierenden Magnetfeldern ihre Phasenbeziehung verlieren. Gleichzeitig nimmt die Gesamtmagnetisierung in Richtung des Magnetfeldes wieder exponentiell mit der Relaxationszeit T₁ auf die Gleichgewichtsmagnetisierung hin zu. Mit Hilfe von zu richtigen Zeitpunkten geschalteten magnetischen Gradientenfeldern ist es möglich, unterschiedliche Kombinationen aus der Spindichte und den beiden Relaxationszeiten ortsaufgelöst in einem grauwertkodierten Bild darzustellen.

Weiter ist es bekannt, mit Hilfe eines Schwingkreises lokal eine Verstärkung der Anregung von Kernspins herbeizuführen. Hierzu sind sogenannte "Fiducial Markers" bekannt, die spezielle, mit signalintensiven Flüssigkeiten gefüllte Kompartments aufweisen, die von einem Schwingkreis umgeben sind (Burl et al.: "Tuned Fiducial Markers to Identify Body Locations with Minimal Perturbation of Tissue Magnetization", in: Journal of Magnetic Resonance in Medicine 1996, 491-493). Der Schwingkreis besitzt dabei die Resonanzfrequenz des MR-Systems.

Wird ein solcher Fiducial Marker in das bildgebende Volumen eines Kernspintomographen eingebracht, so wird im Falle der Einstrahlung von elektromagnetischer Strahlung mit der Resonanzfrequenz der Schwingkreis angeregt. Dies führt zu einer Verstärkung des magnetischen Wechselfeldes innerhalb der Induktivität des Schwingkreises. Die erhöhte magnetische Wechselfeldkomponente vergrößert den Drehwinkel α der Protonen innerhalb der Induktivität. Bei einem kleinen Anregungswinkel (α < 90°) der Protonen durch das Kernspinsystem erfahren die Protonen innerhalb der Induktivität einen vergrößerten Anregungswinkel. Im Idealfall werden Protonen im bildgebenden Volumen mit einem kleinen Winkel von 1° - 10° angeregt, wogegen die Protonen innerhalb der Induktivtät mit 90° angeregt werden. Selbst bei identischen Relaxationszeiten und bei einer identischen Spindichte ist das Signal des vom Schwingkreis umgebenen Kompartments deutlich intensiver als das Signal der anderen Bildanteile. Da diese Signalanhebung lokalisiert ist, kann sie zur Positionsbestimmung verwandt werden.

Ebenso gilt nach dem Reziprozitätsgesetz, daß die MR-Antwortsignale der Protonen innerhalb des vom Schwingkreis umgebenden Kompartments (Fiducial Markers) verstärkt werden. Durch die Induktivität werden die Magnetfeldlinien, die von den Spins innerhalb der Spule herrühren, gebündelt, so daß mehr Signal aus dem Volumen innerhalb der Induktivität abgestrahlt und in eine zugeordneten Empfangsspule eingestrahlt wird. Diese Verstärkung der abgestrahlten und dann empfangenen Signale ist unabhängig von einer verstärkten Anregung zu sehen. Beide Effekte führen zu einer veränderten Signalantwort des Fiducial Markers.

Nachteilig verwenden "Fiducial Markers" gesonderte signalgebende Volumina, die zur Sichtbarkeit im MR-Bild eine Größe von zumindest einigen Kubikmillimetern aufweisen und im Untersuchungsobjekt extra plaziert oder in die Systeme integriert werden müssen, die im Untersuchungsobjekt plaziert werden. Dies ist häufig nicht möglich.

Mit Einführung offener Magnete und neuer Techniken bei geschlossenen MR-Systemen ist es inzwischen möglich, interventionelle und minimalinvasive Techniken wie Punktion, Katheterisierung und operative Verfahren unter MR-tomographischer Kontrolle durchzuführen. Dabei führen ferromagnetische oder paramagnetische Metalle oder Verunreinigungen anderer Werkstoffe zu Artefakten in der Bilddarstellung.

Bei den für interventionelle und minimalinvasive Techniken verwendeten Geräten ergeben sich dabei insofern Probleme, als sie meist aus einem ferromagnetischen oder paramagnetischen Material bestehen und/oder derart klein sind, daß sie in der Größenordnung der Pixelgröße (ca. 1mm) der MR-Bilder liegen. Insbesondere Stents aus Metall oder Kunststoffen sind wegen ihrer feinen Struktur im MR-Bild kaum sichtbar und höchstens durch Artefakte lokalisierbar. Sofern im MR-Bild nicht sichtbare Stoffe verwendet werden, sind diese nur als "Schatten" erkennbar. Diese Nachteile führen dazu, daß eine MR-Überwachung häufig nur unbefriedigend ist und stattdessen ein röntgenologisches Verfahren zur Bilddarstellung verwendet wird, mit den bekannten Nachteilen derartiger Verfahren.

Aus der DE 195 10 194 A1 ist ein aktiv-invasives Magnetresonanzsystem zur Erzeugung selektiver MR-Angiogramme bekannt, bei dem ein Invasiv-Gerät mit einer HF-Spule versehen ist, mit deren Hilfe lokal die Kernspin-Magnetisierung des in dem Gefäß fließenden Blutes verändert wird. Mit speziellen MR-Bildimpulssequenzen wird selektiv nur das Blut erfaßt und dargestellt, das eine veränderte Kernspin-Magnetisierung aufweist.

Die US-A- 5,445,151 beschreibt ein Verfahren zur Flußmessung strömender Flüssigkeiten, insbesondere von Blut, bei dem an einem Invasiv-Gerät mindestens zwei HF-Spulen vergesehen sind, wobei eine von der einen HF-Spule erzeugte lokale Veränderung der Kernspin-Magnetisierung an der anderen HF-Spule erfaßt und die Verzögerungszeit zur Bestimmung der Flußgeschwindigkeit ausgewertet wird.

Die beiden vorgenannten Druckschriften betreffen nicht die Darstellung in einen Körper eingebrachter medizinischer Vorrichtungen. Des weiteren haben sie den Nachteil, daß sie aktive Systeme darstellen, bei denen die eingeführten Geräte ständig über Kabelverbindungen mit extrakorporalen Komponenten in Verbindung stehen.

Die DE 195 07 617 A1 beschreibt ein MR-Verfahren, bei dem in ein Untersuchungsobjekt ein chirurgisches Instrument, etwa ein Katheter, eingeführt wird, an dessen Spitze eine Mikrospule befestigt ist. Die Position der Mikrospule wird unter Verwendung bestimmter Sequenztechniken bestimmt.

Aus der EP-A-0 768 539 ist ein MR-Verfahren zur Bestimmung der Position eines Objektes bekannt, das in den Körper eines Patienten eingebracht wird. Dabei wird eine Spulenanordnung ohne Verbindung mit extrakorporalen Komponenten an dem in den Körper einzuführenden Objekt, etwa einem Kathether oder einem chirurgischen Instrument, befestigt und eine aufgrund der Spule erfolgte Signaländerung zur Lokalisation des Objektes verwendet.

### Aufgabe der Erfindung

Der Erfindung liegt die Aufgabe zu Grunde, ein MR-Bildgebungsverfahren zur Darstellung und Positionsbestimmung eines in ein Untersuchungsobjekt eingeführten Stents sowie einen Stent zur Verfügung zu stellen, die eine deutliche und signalintensive Darstellung eines Stents im MR-Bild sowie eine verbesserte Flußmessung ermöglichen.

### Zusammenfassung der Erfindung

Diese Aufgabe wird erfindungsgemäß durch ein MR-Bildgebungsverfahren mit den Merkmalen des Anspruchs 1 und einen Stent mit den Merkmalen des Anspruchs 8 gelöst. Vorteilhafte und bevorzugte Ausgestaltungen der Erfindung sind in den Unteransprüchen angegeben.

Die erfindungsgemäße Lösung sieht vor, in das Gerüst des in das Untersuchungsobjekt einzuführenden Stents einen Schwingkreis zu integrieren bzw. durch das Gerüst auszubilden, der in einem lokal begrenzten Bereich in oder um den Stent eine veränderte Signalantwort erzeugt, die ortsaufgelöst dargestellt wird. Die Resonanzfrequenz des Schwingkreises ist dabei im wesentlichen gleich der Resonanzfrequenz der eingestrahlten hochfrequenten Strahlung des MR-Bildgebungssystems. Über den im MR-Bild entsprechend hervorgehobenen Bereich ist die Position des Stents deutlich feststellbar, da dieser Bereich unmittelbar von innen oder außen an den Stent angrenzt. Da eine veränderte Signalantwort des zu untersuchenden Objekts selbst erzeugt wird, treten dabei nur solche Artefakte auf, wie sie durch das Material des Stents erzeugt werden.

Durch die deutliche Darstellung des Stents im MR-Bild ist eine genaue Positionsbestimmung möglich. Aufgrund der veränderten Signalverhältnisse ist darüber hinaus auch eine verbesserte Flußmessung des durch den Stent oder am Stent vorbei strömenden Mediums möglich. Dabei wird ausgenutzt, daß innerhalb und außerhalb des Stents eine unterschiedliche Anregung vorliegt.

Die erfindungsgemäße Lösung beruht auf der überraschenden Erkenntnis, daß geeignete Schwingkreise an einem Stent selbst ausgebildet werden können. Die Erfindung sieht dabei vor, daß die den Schwingkreis bildende Induktivität und Kapazität durch das Material des Stents ausgebildet wird, wodurch ein synergistischer Effekt entsteht. Alternativ werden Induktivität und Kapazität als gesonderte Bauteile in den Stent integriert, wobei die Induktivität durch eine gesonderte Spule gebildet wird, die derart in das auffaltbare Gerüst des Stents eingeflochten ist, daß sie sich beim Entfalten des Stents zusammen mit dem Gerüst auffaltet.

Die Signalantwort der Spins innerhalb der Induktivität wird erfindungsgemäß verändert. Hierzu tragen zwei Vorgänge bei. Zum einen wird bei Einstrahlung der hochfrequenten Strahlung der auf die Resonanzfrequenz abgestimmte Schwingkreis angeregt und erfahren die vom Feld des Schwingkreises erfaßten Kernspins eine verstärkte Anregung durch eine lokale Verstärkung des wechselnden magnetischen Feldes in oder in der Nähe der Induktivität. Mit anderen Worten werden von den Feldlinien des induzierten Magnetfeldes erfaßte Protonen um einen größeren Winkel gedreht als Protonen außerhalb dieses induzierten Magnetfeldes. Es erfolgt ein verstärktes Umkippen der Kernspins. Entsprechend kann die von einer Empfangsspule erfaßte und zur Bilddarstellung ausgewertete Signalantwort verstärkt sein. Ebenfalls ist möglich, daß nur die Spins innerhalb der Induktivität eine Sättigung erfahren und das Signal gegenüber der Umgebung abgeschwächt ist. In beiden Fällen liegt eine Änderung der Signalantwort vor.

Zum anderen werden - unabhängig von einer verstärkten Anregung - die MR-Antwortsignale der Protonen innerhalb der Induktivität verstärkt. So werden durch die Induktivität die Magnetfeldlinien, die von den Spins innerhalb der Induktivität herrühren, gebündelt, so daß mehr Signal abgestrahlt und in eine zugeordneten Empfangsspule eingestrahlt wird, die die verstärkten Signale empfängt und zur MR-Bildgebung weiterleitet. Dieser Effekt ist in der Veröffentlichung J. Tanttu: "Floating Surface Coils", in: XIV ICMBE AND VII ICMP, Espoo, Finland 1985" beschrieben.

Diese beiden Effekte können bei dem erfindungsgemäßen Verfahren beide zur Veränderung der Signalantwort genutzt werden. Es kann jedoch auch allein der zweite Effekt, d.h. eine Verstärkung der MR-Antwortsignale, ausgewertet werden.

Dementsprechend ist eine erste Ausgestaltung der Erfindung
dadurch gekennzeichnet, daß bei Einstrahlung der hochfrequenten Strahlung der Schwingkreis angeregt und dadurch in dem lokal begrenzten Bereich eine verstärkte Anregung der Kernspins des Untersuchungsobjekts erfolgt. Bevorzugt liegt der lokal begrenzte Bereich, in dem eine Verstärkung der Anregung der Kernspins erfolgt, dabei innerhalb des Stents. Dies ist natürlicherweise der Fall, wenn das Gerüst des Stents die Induktivität ausbildet.

Eine zweite Ausgestaltung der Erfindung sieht dagegen vor, daß bei Einstrahlung der hochfrequenten Strahlung der Schwingkreis verstimmt oder die Kapazität kurzgeschlossen wird, so daß keine verstärkte Anregung der Kernspins in dem lokal begrenzten Bereich erfolgt. Bei Messung der Signalantwort des lokal begrenzten Bereichs wird die Verstimmung des Schwingkreises bzw. der Kurzschluß der Kapazität jedoch wieder aufgehoben, so daß der Schwingkreis eine Verstärkung der abgestrahlten MR-Antwortsignale der Protonen bewirkt. Es hat sich gezeigt, daß insbesondere diese Variante es ermöglicht, mit hoher Qualität den Bereich in und um den Stent abzubilden, d.h. über eine reine Positionsbestimmung hinaus eine lokale Bildgebung zur Verfügung stellt. Neben der Position des Stents sind dem MR-Bild verbesserte Aussagen über die Struktur etc. des Inneren und/oder der Umgebung des Stents zu entnehmen.

Eine Verstärkung der Anregung der Kernspins wird beispielsweise dadurch unterdrückt, daß der Kondensator des Schwingkreises durch gekreuzte Dioden bei der Anregung kurzgeschlossen wird. Die Verstärkung der abgestrahlten Signale wird dadurch nicht beeinflußt, da die geringe induzierte Spannung durch die innerhalb der Induktivität befindlichen Spins bei der Abstrahlung unterhalb der Durchlaßspannung der Dioden liegt.

Es wird allgemein darauf hingewiesen, daß die erfindungsgemäße Änderung der Signalantwort meist eine Verstärkung der Signalantwort sein wird. Dies hängt jedoch von zahlreichen Faktoren ab, insbesondere den verwendeten Anregungssequenzen. Beispielsweise kann bei schnell aufeinanderfolgenden Sequenzen eine Sättigung der Anregung der Spins innerhalb der Induktivität vorliegen, so daß dort kein Signal erzeugt wird. In dem Bereich außerhalb der Induktivität, in dem eine geringere Anregung der Kernspins erfolgt, liegt dagegen keine Sättigung vor, so daß hier ein Signal erzeugt wird. Entsprechend erfolgt bei diesem Beispiel eine Verminderung der Signalanwort im vom Feld der Induktivität erfaßten Bereich.

In einer bevorzugten Ausgestaltung der Erfindung ist vorgesehen, daß der Schwingkreis nach Einbringen des Stents in das Untersuchungsobjekt durch Auffalten des Stents auf die Resonanzfrequenz eingestellt wird.

Mit Vorteil sind zur resonanten Abstimmung des Schwingkreises die Induktivität und/oder die Kapazität einstellbar. Dies ist insbesondere für den Fall sinnvoll, daß sich nach Applikation bzw. Auffalten des Stents das Produkt von Induktivität und Kapazität und somit die Resonanzfrequenz des Schwingkreises ändern.

In einer vorteilhaften Ausführungsform der Erfindung werden mindestens zwei am Stent ausgebildete oder angeordnete Schwingkreise verwendet, wobei die Spulen der jeweiligen Induktivitäten insbesondere senkrecht zueinander ausgerichtet oder hintereinander angeordnet sind. Durch senkrecht zueinander angeordnete Spulen wird sichergestellt, das bei jeder Anordnung des Stents im äußeren Magnetfeld eine Komponente der Induktivität senkrecht zur Feldrichtung des äußeren Magnetfelds verläuft, so daß eine veränderte Signalantwort sichergestellt wird. Hintereinander angeordnete Spulen sind besonders geeignet, unter Verwendung geeigneter Sequenztechniken zusätzlich eine Flußmessung (d.h. Geschwindigkeitsbestimmung) des den Stent durchfließenden oder an diesem vorbeiströmenden Mediums durchzuführen.

Für das MR-Bildgebungssystem können beliebige konventionelle Systeme verwendet werden.

Bei dem erfindungsgemäßen Stent wird die Induktivität in einer ersten Alternative durch das Material bzw. Gerüst des Stents ausgebildet. Hierdurch werden zusätzliche Teile vermieden und wird die Induktivität auf einfache Weise und automatisch bei Auffalten des Stents während der Applikation ausgebildet.

Der Stent besteht dazu bevorzugt aus einem Material, das mindestens eine Schicht mit guter Leitfähigkeit aufweist, die die Induktivität ausbildet, und eine weitere Schicht mit schlechter Leitfähigkeit, die das Gerüst für die eigentliche Stentfunktion bildet. Die Schicht guter Leitfähigkeit ist an geeigneten Stellen durchtrennt, so daß verschiedene gegeneinander isolierte Bereiche des Gerüsts entstehen, die eine Induktivität ausbilden.

Alternativ wird die Induktivität des Schwingkreises durch eine gesonderte Spule gebildet, die in den Stentaufbau integriert ist. Beispielsweise ist die Spule in das Gerüst des Stents gewebt, gestrickt, geschweißt, gelötet oder geklebt. Die Spule ist dabei derart mit dem Gerüst verbunden, daß sie sich beim Entfalten des Stents zusammen mit dem Gerüst entweder selbstexpandierend elastisch, fremdexpandierend plastisch oder thermisch induziert auffaltet.

Das Gerüst des Stents liegt bevorzugt in Form einer Helix, einer Doppel- oder Mehrfachhelix, als Metallgerüst, oder als gestricktes, geschnittenes oder geäztes Blech oder Rohr vor.

Die Kapazität des Stents ist bevorzugt zumindest teilweise ebenfalls aus dem Stent-Material gebildet, insbesondere durch parallele Schlaufen der Induktivität, wobei zwischen den Schlaufen ein Dielektrikum angeordnet ist. Die Kapazität kann allgemein durch geeignete Anordnungen der leitenden Schichten und einem Material wie dem Stentgerüst als Dielekrikum gebildet werden. Bei entsprechender Geometrie der den Kondensator bildenden Elemente kann auch das Implantatgewebe als Dielektrikum dienen.

Alternativ ist vorgesehen, die Kapazität des Stents durch einen gesondert vorgesehenen Kondensator zu bilden, der mit dem Stentkörper verbunden ist.

Mit Vorteil ist die erfindungsgemäße Vorrichtung derart ausgebildet ist, daß bei einer Veränderung der Geometrie der Vorrichtung durch die Applikation, etwa bei einer Aufweitung eines Stents, das Produkt aus Induktivität und Kapazität des Schwingkreises im wesentlichen konstant bleibt, insbesondere eine Erhöhung des Induktivität mit einer Verringerung der Kapazität oder umgekehrt einhergeht. Hierdurch wird gewährleistet, daß die Resonanzfrequenz im wesentlichen unverändert bleibt.

### Beschreibung mehrerer Ausführungsbeispiele

Die Erfindung wird nachfolgend unter Bezugnahme auf die Figuren der Zeichnung an mehreren Ausführungsbeispielen näher erläutert. Es zeigen:
- Fig. 1 -: schematisch einen erfindungsgemäßen Stent, der einen Schwingkreis mit einer Induktivität und einer Kapazität ausbildet;
- Fig. 2a-2g -: verschiedene elektrische Schaltbilder einer erfindungsgemäßen Vorrichtung;
- Fig. 3 -: eine genauere Darstellung des Gerüsts des Stents der Fig. 1;
- Fig. 4a-4b -: zwei Beispiele für den Aufbau des Stentmaterials;
- Fig. 5 -: einen Schnitt durch das Stentmaterial der Fig. 4a;
- Fig. 6 -: schematisch einen erfindungsgemäßen Stent mit integrierter Spule;
- Fig. 7 -: einen erfindungsgemäßen Stent, der eine zweite, senkrecht zur ersten Induktivität ausgebildete Induktivität aufweist und
- Fig. 8 -: einen erfindungsgemäßen Stent mit zwei hintereinander angeordneten Schwingkreisen.

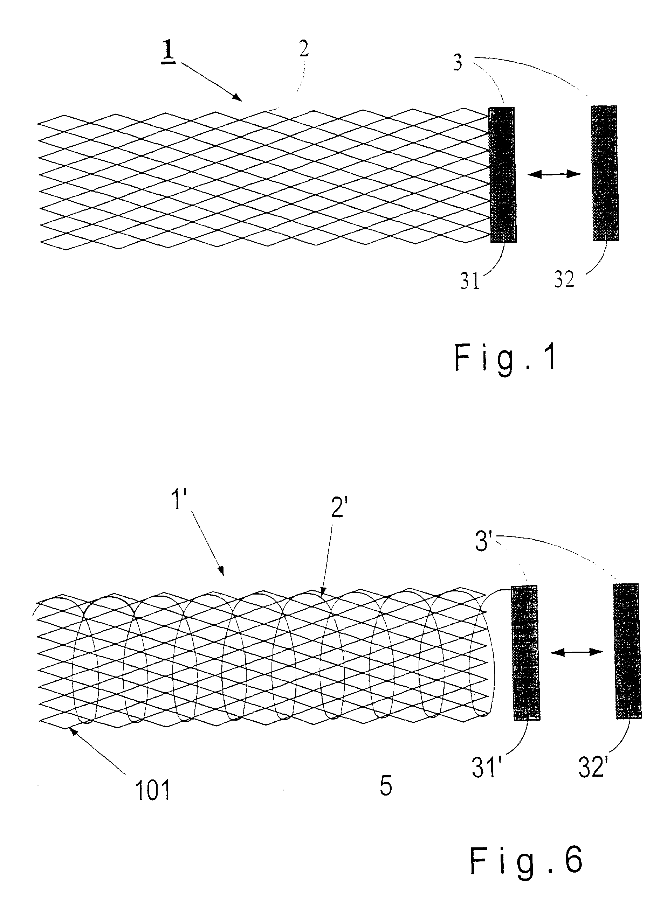

Figur 1 zeigt schematisch einen erfindungsgemäßen Stent 1, der beispielsweise aus Metall wie Platin, Titan oder Titan-Legierungen und -verbindungen, oder aus Kunststoff oder Kohlenstoffaser besteht. Das Einsatzgebiet von Stents liegt insbesondere in der Überbrückung von tumor- oder anders bedingten Engstellen (z.B. Gastrointestional und Bronchialtrakt) bei inneren Organen, arteriellen und venösen Gefäßverengungen, peripheren und zentralen Gefäßstenosen, insbesondere bei der koronaren Herzerkrankung. Weitere Anwendungen sind die Schaffung neuer Gefäßwege (Shunts) in Organen, z.B. in der Leber.

Der Stent führt dabei zu einer mechanischen Versiegelung und dauerhaften Erweiterung der betroffenen Region, hinterläßt eine glatte Fläche mit verbesserter Blutströmung, vergrößert das Gefäßvolumen und vermindert den Wiederverschluß, der nach einer konventionellen Ballondilatation häufiger auftritt.

Die Erfolgsquote von Stents ist jedoch eingeschränkt, da im Bereich des Gerüstes Wiederverschlüsse, z. B. durch Einwachsen von Tumorgewebe oder durch Blutgerinsel, sogenannte Thrombosen, entstehen können, die das Lumen akut verschließen. Durch wucherndes, das Gerüst berührendes Gewebe können neue arteriosklerotische Ablagerungen entstehen und das Lumen des Stents wiederverengen oder verschließen. Da klinische Symptome erst bei höhergradigen Verengungen entstehen, die aber je nach Grad der Enge ein immer höheres Risiko des akuten Verschlusses durch Thrombosen haben, ist die Nachkontrolle der Stentfunktion sehr wichtig. Dies ist bis heute jedoch nur mit einer invasiven Katheterisierung des betroffenen Gefäßes, Gabe von allergenem nierenbelastenden Kontrasmitteln und der Röntgenstrahlung möglich. Aufgrund der ausgeprägten Suszeptibilitätsartefakte von herkömmlichen Stents ist eine MR Kontrolle kaum möglich. Ultraschalluntersuchungen sind durch das ausgeprägte Schallechobildung an dem Stentgerüst ebenfalls sehr eingeschränkt. Das Innere des Stentes entzieht sich bisher jeder Form von medizinisch diagnostischer Bildgebung.

Stents bestehen üblicherweise aus Metallgerüsten, z.B. fortlaufenden Metalldrähten oder einer Art Geflechtschlauch oder werden aus Metallrohren unter Verwendung von Laser- oder Funkenerosionstechniken hergestellt. Es wird im Rahmen dieser Anmeldung für alle diese Stentausgestaltungen der Begriff "Gerüst" verwendet. Zur Applikation wird ein Stent beispielsweise auf einen Ballon-Katheter aufgelegt, mittels des Katheters am Ort der Implantation plaziert und dann entfaltet, wobei der Ballon den Durchmesser des Stents vergrößert und sich dieser gegen die Gefäßwand drückt. Neben ballonexpandierenden plastisch verformbaren Stents sind selbstexpandierende elastische oder thermisch expandierende Stents bekannt.

Die bekannten Stents sind aufgrund ihrer metallischen Struktur in einem MR-Bild nicht darstellbar, sondern bilden teilweise ausgeprägte Artefakte, so daß eine genaue Plazierung und Überwachung der Plazierung im zeitlichen Verlauf sowie eine funktionelle Kontrolle nach der Plazierung bei Verwendung der Magnetresonanztomographie als bildgebendem Verfahren nicht gewährleistet sind.

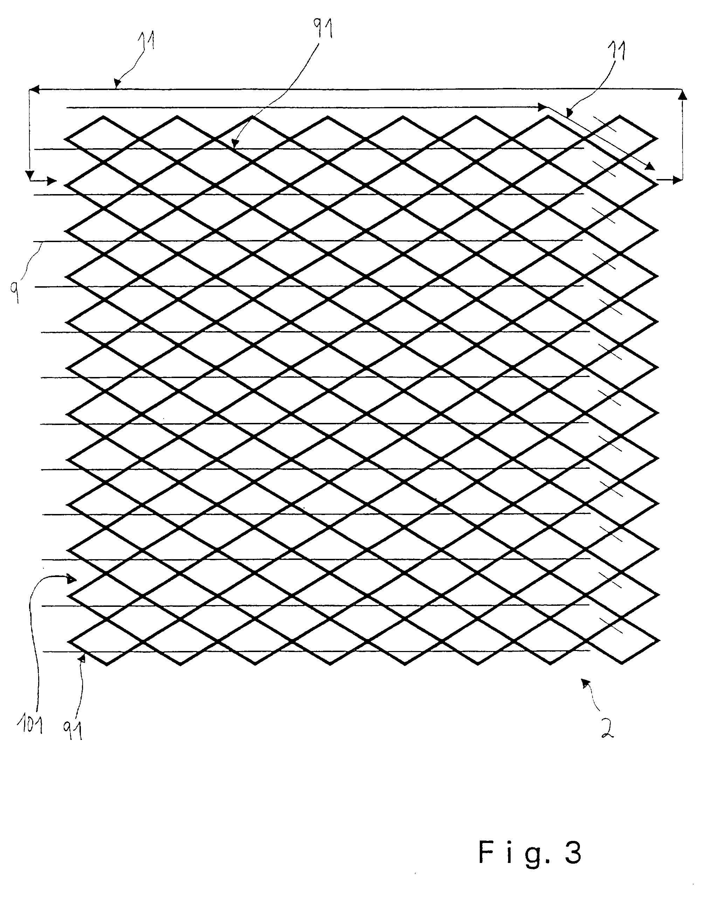

Für eine verbesserte Darstellung und Funktionskontrolle des Stents im MR-Bild weist der erfindungsgemäße Stent 1 gemäß Figur 1 eine Induktivität 2 und eine Kapazität 3 auf. Die Induktivität des Stents 1 wird dabei durch das Gerüst 2 des Stents 1 gebildet. Hierzu ist vorgesehen, daß die einzelnen Komponenten des Gerüsts 2 zueinander isoliert werden, wie anhand der Figur 3 noch im einzelnen beispielhaft aufgezeigt werden wird. Eine Isolierung der einzelnen Komponenten des Gerüstes kann dabei bereits während des Herstellungsverfahrens erfolgen, wobei zwischen den einzelnen Phasen der Herstellung des Stents aus einem Metallrohr jeweils eine Isolierschicht auf das bereits ausgebildete Gerüst aufgetragen wird.

Die Induktivität 2 ist mit einer Kapazität 3 elektrisch verbunden, wobei Induktivität 2 und Kapazität 3 einen Schwingkreis bilden. Der Kondensator 3 ist in Figur 1 als Plattenkondensator mit zwei Platten 31, 32 ausgebildet. Es kann jedoch auch ein beliebig anderer Kondensator verwendet werden. Dabei liegt es auch im Rahmen der Erfindung, daß der Kondensator 3 kein eigenes Bauelement darstellt, sondern ebenso wie die Induktivität 2 aus dem Material des Stents 1 besteht, beispielsweise durch parallele Drähte des Drahtgerüsts gebildet wird. Es sei angemerkt, daß die elektrische Verbindung zwischen der Kondensatorplatte 32 und der Induktivität 2 in Figur 1 für eine bessere Übersichtlichkeit der Darstellung nicht dargestellt ist.

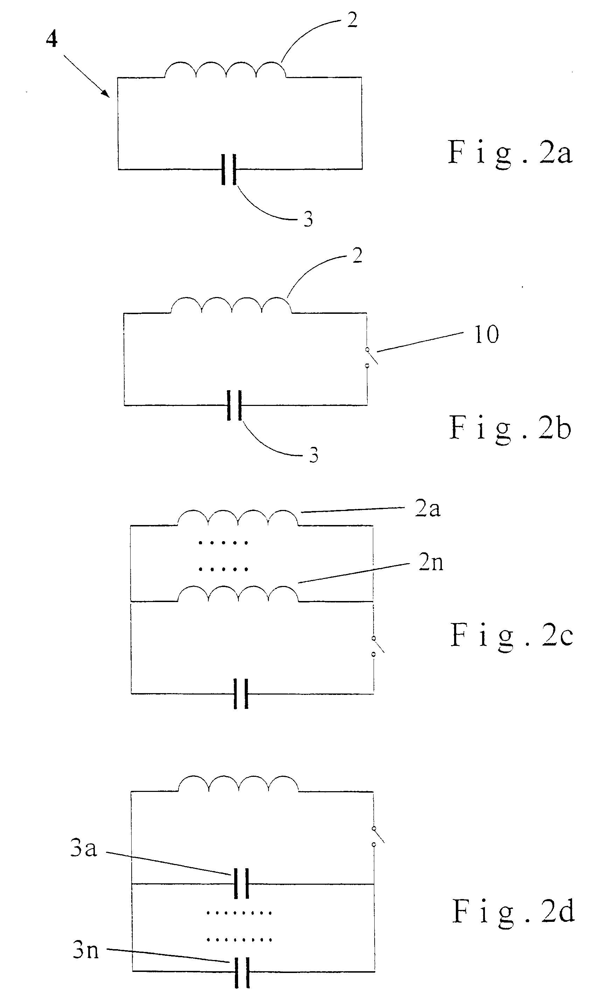

In Figur 2a ist das elektrische Schaltbild des im Stent 1 ausgebildeten Schwingkreises 4, bestehend aus Induktivität 2 und Kapazität 3, dargestellt. Optional ist gemäß Fig. 2b zusätzlich ein Schalter 10 vorgesehen, der mechanisch, etwa durch einen zur Applikation dienenden Katheter, elektrisch oder magnetisch aktivierbar bzw. deaktivierbar ist.

Der Schwingkreis 4 kann auf vielfältigste Weise ausgebildet sein. Gemäß Fig. 2c kann er mehrere parallel geschaltete Induktivitäten 2a bis 2n und gemäß Fig. 2d mehrere parallel geschaltete Kapazitäten 3a bis 3n aufweisen. Des weiteren können mehrere Induktivitäten und/oder Kapazitäten seriell geschaltet sein. Auch kann vorgesehen sein, an einem Stent mehrere Schwingkreise auszubilden, die jeweils einen Schalter und seriell und/oder parallel geschaltete Induktivitäten und/oder Kapazitäten aufweisen können. Speziell bei mehreren parallel oder seriell geschalteten Induktivitäten können mit Hilfe geeigneter Sequenzen Flußmessungen verfeinert werden.

Der Schwingkreis 4 weist eine Resonanzfrequenz auf, die der Resonanzfrequenz der eingestrahlten hochfrequenten Strahlung des MR-Bildungsgebungssystems entspricht, in dem der menschliche Körper, in den der Stent appliziert wird, angeordnet ist.

Bei dem erfindungsgemäßen Stent 1 wird der Schwingkreis 4 durch die eingestrahlten hochfrequenten Pulse des MR-Systems angeregt, da seine Resonanzfrequenz der Frequenz der eingestrahlten HF-Pulse entspricht. Dies führt zu einer Verstärkung des Magnetfeldes in der Induktivität des Schwingkreises oder in der Nähe der Induktivität, was wiederum zu einer verstärkten Anregung der Protonen im entsprechenden Bereich führen kann. Bei einer Anregung der Kerne außerhalb der Induktivität um einen Winkel, der kleiner als 90° ist, können Kerne innerhalb der Induktivität eine Anregung von 90° erfahren und so mit maximaler Amplitude antworten. Die im Bereich der Induktivität angeordneten Protonen bzw. Kerne erfahren somit eine stärkere Anregung, als außerhalb der Induktivität angeordneten Protonen.

Die Verstärkung des Drehwinkels innerhalb der Induktivität kann gegenüber den Protonen außerhalb der Induktivität einen Faktor von bis zu 45 betragen. Es ist daher möglich, die Protonen im Inneren der Induktivität um einen Winkel von 90° auszulenken (maximale Signalantwort), während die Protonen außerhalb der Induktivität bzw. außerhalb des durch den Schwingkreis erzeugten magnetischen Feldes lediglich eine Kleinwinkelanregung von ca. 2° bis 10° erfahren. Dies führt dazu, daß der innere Bereich des Stents in einem MR-Bild wesentlich heller dargestellt werden kann als die restliche Umgebung. Es kann daher die Lokalisation des Stents im menschlichen Körper genau bestimmt werden.

Zur Abstimmung der Resonanzfrequenz des Schwingkreises 4 auf die Frequenz der eingestrahlten HF-Pulse sind verschiedene Gestaltungen des Schwingkreises 4 möglich.

In einer Variante ist vorgesehen, daß die Güte des Schwingkreises relativ gering gehalten wird, um einen möglichst breitbandigen Schwingkreis zu realisieren und einen möglichst großen Bereich von Resonanzfrequenzen abzudecken.

Eine zweite Variante sieht vor, die Vorrichtung so auszubilden, daß auch nach einer Änderung der Geometrie, im betrachteten Beispiel nach dem Entfalten des Stents, das Produkt aus Induktivität und Kapazität konstant ist. Dies kann entweder dadurch erfolgen, daß dem Stent eine Geometrie gegeben wird, die bei einer Entfaltung des Stents ihre Eigenschaften möglichst wenig ändert, also insbesondere eine konstante Induktivität und eine konstante Kapazität aufweist. Eine Aufweitung des Stents am Ort der Implatierung bewirkt somit im wesentlichen keine Änderung der Resonanzfrequenz des Schwingkreises.

Eine Konstanz des Produktes von Induktivität und Kapazität kann zum anderen durch eine Kompensierung der sich ändernden Induktivität durch eine sich entsprechend ändernde Kapazität verwirklicht werden. Zur Kompensierung einer sich ändernden Induktivität durch eine sich entsprechend ändernde Kapazität ist beispielsweise vorgesehen, daß eine Kondensatorfläche verschiebbar angeordnet ist, so daß die Kapazität sich entsprechend dem Abstand der Kondensatorflächen vergrößert bzw. verkleinert. Die Verschiebbarkeit der Kondensatorplatte 32 gegenüber der Kondensatorplatte 31 und Einstellbarkeit der Kapazität ist in Figur 1 durch einen Doppelpfeil schematisch dargestellt.

Eine dritte Variante sieht vor, daß eine Anpassung des Schwingkreises im Magnetfeld des Kernspintomographen durch eine Veränderung bzw. Einstellung der Induktivität und/oder Kapazität des Schwingkreises nach deren Plazierung erfolgt. Hierzu ist beispielsweise vorgesehen, die Kondensatorfläche mit Hilfe des noch im Körper befindlichen Applikationsinstruments, etwa Katheters, zu verändern. Eine Verminderung der Induktivität und damit eine Anpassung des Schwingkreises auf die Resonanzfrequenz im Kernspintomographen kann etwa durch eine laserinduzierte, mechanische oder elektrolytische Isolierung von Spulensegmenten erfolgen. Eine Änderung der Kapazität kann ebenso durch eine laserinduzierte, mechanische oder elektrolytische Isolierung von Kapazitäten erfolgen.

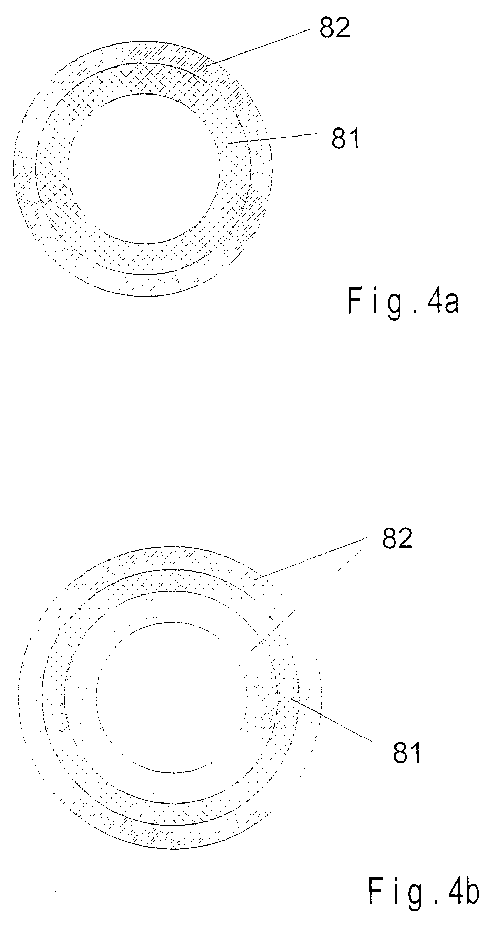

Fig. 3 zeigt schematisch eine mögliche Ausgestaltung eines Stents gemäß der Figur 1. Das Stentmaterial besteht dabei gemäß Fig. 4a und 4b aus zwei oder mehr Schichten 81, 82. Die eine Schicht 81 stellt das Material für die eigentliche Stentfunktion dar. Sie weist eine schlechte Leitfähigkeit und eine hohe Stabilität und Elastizität auf. Als Materialien kommen insbesondere Nickel-Titan, Kunststoff oder Kohlenstofffasern in Frage. Die Schicht(en) 82 stellen das Material zur Ausbildung der Induktivität zur Verfügung. Die Schicht 82 weist hierzu eine sehr gute Leitfähigkeit auf. Als Materialen kommen insbesondere Gold, Silber oder Platin in Frage, die sich neben einer hohen Leitfähigkeit durch Biokompatibilität auszeichnen. Bei Verwendung weniger biokompatibler elektrischer Leiter wie Kupfer kann durch eine geeignete Kunststoff- oder Keramikbeschichtung elektrische Isolation und Biokompatibilität erreicht werden.

Die Herstellung des Stentmaterials gemäß Figuren 4a, 4b erfolgt zum Beispiel dadurch, daß ein Rohr aus Titan oder Titan-Legierungen oder -verbindungen mit dem Material für die Ausbildung der Induktivität beschichtet und anschließend durch an sich bekannte Laser- oder Funkenerosion- oder Wasserstrahl-Schneidetechniken geschnitten wird.

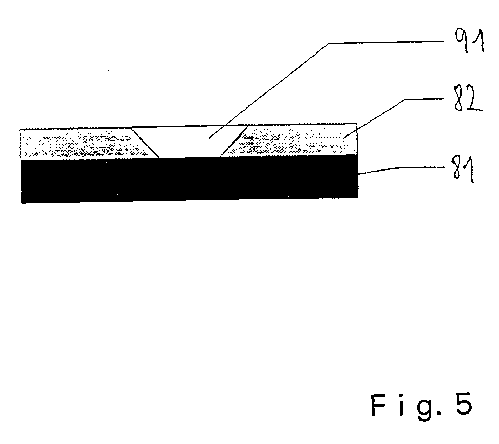

Eine Spule mit dem Material der Fig. 4a wird gemäß Fig. 3 wie folgt ausgebildet. Der Stent 1 besteht aus einem zweilagigen Material, das eine wabenförmige Struktur 101 ausbildet und etwa aus einem Rohr durch Laser-Schneidetechniken herausgearbeitet wird. Fig. 3 zeigt das Rohr auseinandergeklappt. Die rechte und linke Seite sind daher identisch. Die leitfähige Schicht der wabenförmigen Struktur ist entlang der Linien 9 unterbrochen. An den entsprechenden Stellen 91 wird die leitfähige Schicht hierzu bei der Herstellung des Stents nach Ausbildung der Struktur mit Hilfe eines chemischen, physikalischen oder mechanischen Verfahrens durchtrennt. Eine Stelle 91, in der die auf dem eigentlichen Stentmaterial 81 angeordnete leitfähige Schicht 82 unterbrochen ist, ist in Figur 5 schematisch dargestellt.

Durch die Trennstellen 91 wird ein Stromweg durch das leitfähige Material 82 definiert, der in Fig. 3 durch Pfeile 11 angedeutet ist. Es entsteht eine Spulenanordnung 2, die die Induktivität des Stents 1 darstellt. Zur Spulenfunktion ist das leitfähige Material derart zu wählen, daß der Widerstand durch den aus dem leitfähigen Material gebildeten Leiter von einem Ende des Stents zum anderen geringer ist als der durch das Stentmaterial vorgegebene Widerstand.

Die Induktivität 2 bildet sich bei einem Auffalten des Stentmaterials während der Applikation des Stents automatisch aus.

Bei Verwendung eines dreilagigen Materials entsprechend Figur 4b erfolgt die Ausbildung einer Induktivität in entsprechender Weise, wobei dann beide Schichten des leitfähigen Materials Trennstellen zur Ausbildung eines Stromwegs aufweisen. Die Verwendung zweier leitfähiger Schichten weist den Vorteil auf, daß sich der Querschnitt der Leiterbahnen effektiv verdoppelt.

In einer Weiterbildung des Ausführungsbeispiels der Fig. 3 bis 5 ist die leitfähige Schicht 82 jeweils zusätzlich nach außen mit einem isolierenden Kunststoff wie einem Pyrolen beschichtet, um einen Stromfluß durch das angrenzende Blut sicher zu verhindern, der die Induktivität der Spule herabsetzen würde. Pyrolene sind geeignet, da sie biokompatibel sind und sich recht gut mit Metall-Legierungen verbinden. Zum Beschichten mit Pyrolenen wird der Stent nach seiner Herstellung beispielsweise in ein Bad mit Pyrolenen gehalten oder mit Pyrolenen bedampft.

Es folgt zur weiteren Veranschaulichung der Erfindung eine Abschätzung der erforderlichen Kapazitäten und Induktivitäten. In dem Beispiel wird ein Plattenkondensator verwendet und die Spule wird als Helix mit einer festen Windungszahl angenommen. Die Resonanzfrequenz eines Kernspinsystems liegt üblicherweise im Bereich zwischen 2 MHz bis 90 MHz. Die Resonanzfrequenz des Kernspinsystems ist dabei gleich dem Produkt aus der magnetischen Feldstärke und dem gyromagnetischen Verhältnis g. Bei einer mittleren Feldstärke von 1 Tesla ergibt sich eine Resonanzfrequenz von ca. 42 MHz. Die Resonanzfrequenz des Schwingkreises ergibt sich aus der Thomsonschen Schwingungsgleichung. Sie ist umgekehrt proportional zur Wurzel aus dem Produkt der Induktivität und der Kapazität.

Das Produkt aus Kapazität und Induktivität ist dann gleich 1,4 x 10⁻¹⁹ S² ist. Bei einem angenommenen Durchmesser des Stents von 8 mm und einer Länge von 40 mm ergibt sich je nach Windungszahl eine Induktivität von etwa 4 x 10⁻⁶ Vs/A. Die sich daraus ergebende resultierende Fläche eines Plattenkondensators bei einer relativen Dielektrizitätskonstanten von 2 und einem Abstand von 0,1 mm der einzelnen Platten des Plattenkondensators beträgt etwa 0,2 mm². Eine derart kleine Fläche eines Plattenkondensators ist in einem Stent leicht zu verwirklichen. Bei höheren Magnetfeldern bzw. Frequenzen läßt sich die resultierende Fläche eines Plattenkondensators auf 0,014 mm² weiter reduzieren.

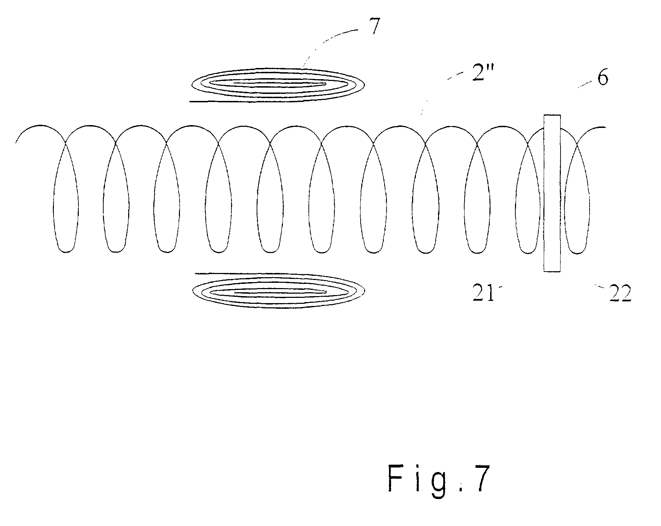

Figur 6 zeigt ein alternatives Ausführungsbeispiel eines Stents 1', der eine Induktivität 2' und eine Kapazität 3' ausbildet. Die Induktivität 2' ist hier in Form einer helixförmigen Spule 5 ausgeführt, die nicht durch das Stentgerüst 101 selbst gebildet wird, sondern in das Stentgerüst 101 als zusätzlicher Draht hineingewebt ist. Die Stentfunktion und die Spulenfunktion sind bei diesem Ausführungsbeispiel getrennt.

Die Spule 5 ist zur Ausbildung eines Schwingkreises wiederum mit einem Kondensator 3' verbunden, der entweder ebenfalls ein gesondertes Bauelement darstellt, oder alternativ durch benachbarte Spulenschleifen oder integrierte Flächen des Stents verwirklicht wird.

Bei Applikation des Stents ist die Spule 5 zusammen mit dem Stentmaterial 101 mit kleinerem Radius auf ein Applikationsinstrument wie einen Kathether aufgewickelt und dehnt sich dann am Ort der Applikation zusammen mit dem Stentmaterial 101 auf den gewünschten Durchmesser auf. Hierzu weist der Draht bzw. die Spule 5 bevorzugt ein Formgedächtnis auf oder wird der Draht bzw. die Spule 5 auf dem Applikationsinstrument vorgespannt.

Die Überschneidungsfläche oder der Abstand der beiden Kondensatorplatten des Kondensators 3' ist zu einer Anpassung der Resonanzfrequenz des Schwingkreises wiederum verschiebbar ausgebildet. Es liegt jedoch durchaus im Rahmen der Erfindung, daß eine Anpassung an die Resonanzfrequenz auf eine andere Art und Weise wie oben beschrieben erfolgt.

Bei dem Ausführungsbeispiel der Figur 7 ist die Induktivität 2'' des Stents schematisch dargestellt. Sie kann entweder aus dem Stentmaterial gebildet (Fig. 3) oder als zusätzlicher Draht ausgeführt sein (Fig. 6). Ein eigener Kondensator ist bei dieser Ausführungsform nicht vorgesehen. Vielmehr bilden zwei Schlaufen 21, 22 der Induktivität 2'' die Kapazität aus, wobei zur Erhöhung der Kapazität zwischen den beiden Schlaufen 21, 22 ein Dielektrikum 6 mit einer möglichst hohen Dielektrizitätskonstante angeordnet ist.

Zusätzlich zu der Induktivität 2'' ist eine weitere Induktivität 7 in Form von einem Spulenpaar 7 vorgesehen, dessen Achse senkrecht zur Achse der Induktivität 2'' liegt. Das Spulenpaar 7 wird beispielsweise durch zwei spiralförmige Spulenanordnungen gebildet, die in das Stentgerüst integriert sind. Hierdurch wird gewährleistet, daß bei jeder Anordnung des Stents im Gewebe eine Komponente senkrecht zur Feldrichtung des homogenen äußeren Magneten vorliegt. Alternativ ist hierzu eine weitere Induktivität senkrecht zu den beiden dargestellten Induktivitäten vorgesehen. Auf diese Weise wird bei jeder Anordnung des Stents im Magnetfeld eine verstärkte Spinanregung im betrachteten Bereich gewährleistet.

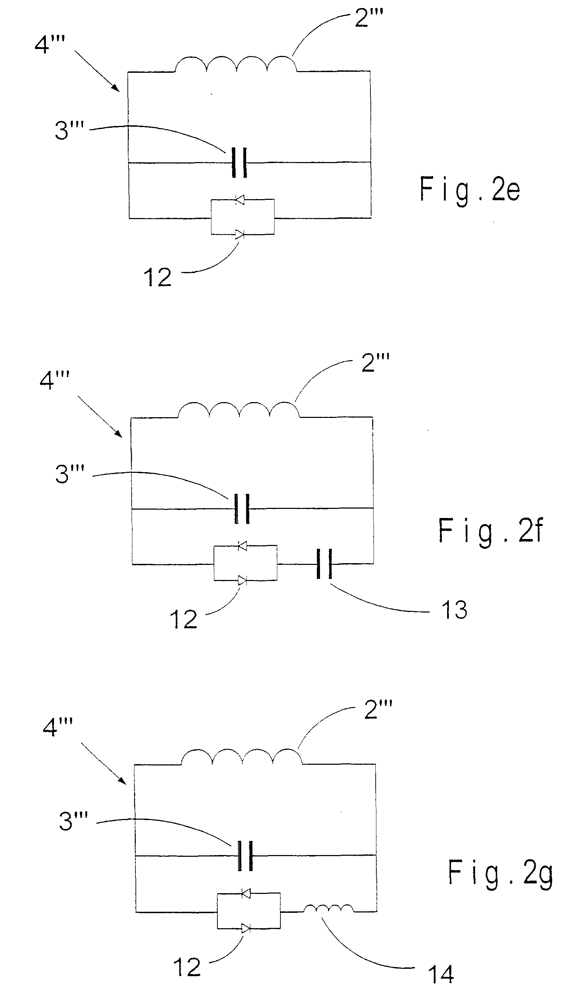

Zwei weitere Varianten der Erfindung sind anhand der Schaltbilder der Figuren 2e und 2f dargestellt. In Figur 2e wird der Kondensator 3''' durch zwei gekreuzte Dioden 12, die als zusätzliche Elemente im Gerüst des Stents enthalten sind, während der Anregungsphase kurzgeschlossen. Die Dioden 12 weisen eine Durchlaßspannung auf, die etwa bei 1 Volt, jedenfalls unterhalb der bei der Einstrahlung hochfrequenter Strahlung erzeugten Spannung liegt, die üblicherweise mehr als 1 Volt beträgt. Die Dioden 12 leiten somit bei Einstrahlung der hochfrequenten Strahlung, so daß der Kondensator 3''' in der Anregungsphase kurzgeschlossen wird und sich kein Schwinkreis ausbildet.

Dies bedeutet, daß bei Einstrahlung der hochfrequenten Strahlung anders als bei den bisherigen Ausführungsbeispielen keine verstärkte lokale Anregung der Kernspins erfolgt. Allerdings wird bei Messung der Signalantwort des von der Induktivität 2''' erfaßten Bereiches der Kurzschluß der Kapazität 3''' wieder aufgehoben. Hierzu sind die Dioden 12 derart ausgestaltet, daß die Durchlaßspannung oberhalb der bei der Spin-Signalantwort erzeugten Spannung liegt. Der Kondensator 3''' wird somit bei Abstrahlung der MR-Antwortsignale der Atomkerne nicht kurzgeschlossen und es entsteht ein Schwingkreis 4 " ', der eine Verstärkung der abgestrahlten MR-Antwortsignale der Protonen bewirkt und dadurch die gemessene Signalantwort verändert.

Die Dioden 12 können auf vielfältige Art im Stentgerüst verwirklicht werden. Insbesondere können gesonderte Bauelemente verwendet oder die Dioden durch oder in Zusammenwirkung mit dem Stentmaterial gebildet werden, etwa als auf das Stentgerüst aufgebrachte Strukturen.

In Fig. 2f wird der Kondensator 3''' bei prinzipiell gleichem Aufbau wie in Fig. 2e nicht kurzgeschlossen, sondern der Schwingkreis 4''' durch Zuschalten eines weiteren Kondensators 13 in der Anregungsphase lediglich verstimmt, so daß eine verstärkte Anregung der Kernspins in nur begrenztem Maße erfolgt. Bei Abstrahlung der MR-Antwortsignale sperren die Dioden 12, so daß der Schwinkreis 4''' dann unverstimmt vorliegt und eine Verstärkung der abgestrahlten MR-Antwortsignale erfolgt, was zu einer veränderten Signalantwort führt, die im MR-Bild dargestellt wird.

In Fig. 2g wird in der Anregungsphase der Schwingkreis 4''' nicht durch Zuschalten eines Kondensators, sondern durch Zuschalten einer Spule 14 verstimmt.

In einer Weiterbildung der Erfindung ist es möglich, auch die Geschwindigkeit des die Vorrichtung durchfließenden Blutes zu bestimmen. Hierzu werden an sich bekannte Sequenztechniken verwendet. Beispielsweise werden im Bereich des blutzuführenden Gewebes vor der Vorrichtung Sättigungsimpulse vorgenommen, wobei eine Variation entweder des Ortes der Sättigungsimpulse oder des Zeitabstandes zwischen Sättigungspuls und Kleinwinkelanregung die Berechnung der Flußgeschwindigkeit und damit funktionelle Aussagen über den Status des Gefäßes ermöglichen. Beliebige bekannte Methoden der Flußbestimmung sind in Verbindung mit dem erfindungsgemäßen Stent einsetzbar. Neue Sequenztechniken können die Eigenschaften des Stents dabei gezielt ausnutzen, d.h. eine verstärkte Anregung und einen verstärkten Empfang oder lediglich einen verstärkten Empfang des vom Stent umschlossenen Bereichs.

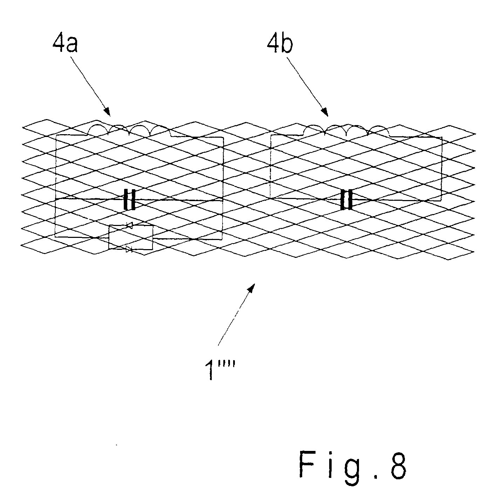

Fig. 8 zeigt einen Stent 1"'', der bevorzugt zur Flußmessung eingesetzt wird. Der Stent weist zwei hintereinander angeordnete Schwingkreise 4a, 4b auf, die schematisch dargestellt sind. Die Schwingkreise 4a, 4b können aus dem Stentmaterial oder durch zusätzliche Bauelemente gebildet sein, wie anhand der obigen Ausführungsbeispiele beschrieben ist. Der eine Schwingkreis 4a weist zwei gekreuzte Dioden entsprechend Figur 2e auf, so daß die Kapazität bei der Anregung kurzgeschlossen wird. Der andere Schwingkreis 4b ist ohne Dioden ausgebildet.

Dies führt dazu, daß bei Einstrahlung hochfrequenter MR-Anregungsimpulse in einem Teilbereich des Stents, nämlich dem Teilbereich, der von dem Schwingkreis 4b ohne Dioden umgeben ist, eine verstärkte Anregung stattfindet. Jedoch liegt auch in dem anderen Teilbereich, der von dem Schwingkreis 4a umgeben ist, eine gegenüber dem umgebenden Gewebe veränderte Signalantwort vor, wie anhand der Figur 2e erläutert wurde. Eine derartige Anordnung ist bei Verwendung geeigneter Sequenztechniken besonders gut zur Flußbestimmung und damit zur funktionellen Kontrolle des Stents geeignet.

In einer Weiterbildung der Erfindung (nicht dargestellt) ist ein Katheter oder Ballon mit einer Empfangspuleneinrichtung ausgerüstet. Statt oder ergänzend zu einer externen Empfangsspule des MR-Systems empfängt der Katheter oder Ballon das vom Stent verstärkte Signal und leitet es extrakorporal weiter. Der Katheter kann hierbei über die gleiche oder ähnliche Anordnung von Induktivität, Kapazität und Dioden verfügen und die Signale des Stentes verstärken und entweder über elektrisch leitende Bahnen oder über optische Koppelung und Glasfasern extrakorporal an den Tomographen weiterleiten. Gegenüber der Verwendung externer Empfangsspulen zeichnet sich diese Variante durch eine verbesserte Signaldetektion aus.

In einer anderen Weiterbildung (nicht dargestellt) der Erfindung ist vorgesehen, daß die Induktivität des Stents auch selbst als Empfangsspule zur Erfassung von MR-Antwortsignalen verwendet wird, wobei die Induktivität dann über eine Kabelverbindung mit extrakorporalen Funktionskomponenten verbunden ist. Hierdurch wird ermöglicht, die Induktivität des Schwingkreises ergänzend aktiv zur Bildgebung zu verwenden. Aufgrund der Notwendigkeit einer Kabelverbindung mit extrakorporalen Funktionskomponenten wird dies jedoch im allgemeinen nur während der Implantation eines Stents in Frage kommen.

Die Erfindung beschränkt sich in ihrer Ausführung nicht auf die vorstehend angegebenen Ausführungsbeispiele. Wesentlich für die Erfindung ist allein, daß der Stent mindestens einen passiven Schwingkreis mit einer Induktivität und einer Kapazität ausbildet oder in sich integriert, derart, daß sich die Induktivität beim Entfalten des Stents zusammen mit dem gerüst auffaltet.

## Patentansprüche

1. MR-Bildgebungsverfahren zur Darstellung und Positionsbestimmung eines in ein Untersuchungsobjekt eingeführten Stents mit auffaltbarem Gerüst, bei dem
a) das Untersuchungsobjekt in einem äußeren Magnetfeld angeordnet,
b) durch Einstrahlung hochfrequenter Strahlung einer bestimmten Resonanzfrequenz Übergänge zwischen Spin-Energieniveaus der Atomkerne des Untersuchungsobjekts angeregt und
c) dabei erzeugte MR-Signale als Signalantwort detektiert, ausgewertet und ortsaufgelöst dargestellt werden,
**dadurch gekennzeichnet,**
daß in einem lokal begrenzten Bereich in oder um den Stent eine veränderte Signalantwort erzeugt wird, indem das Gerüst des Stents mindestens einen Schwingkreis mit einer Induktivität und einer Kapazität ausbildet oder in sich integriert, derart, daß sich die Induktivität beim Entfalten des Stents zusammen mit dem Gerüst auffaltet, wobei die Resonanzfrequenz des Schwingkreises im wesentlichen gleich der Resonanzfrequenz der eingestrahlten hochfrequenten Strahlung ist, und der Bereich mit veränderter Signalantwort ortsaufgelöst dargestellt wird.

2. Verfahren nach Anspruch 1, **dadurch gekennzeichnet,** daß bei Einstrahlung der hochfrequenten Strahlung der Schwingkreis angeregt und dadurch in dem lokal begrenzten Bereich eine verstärkte Anregung der Kernspins des Untersuchungsobjekts erfolgt.

3. Verfahren nach Anspruch 2, **dadurch gekennzeichnet,** daß der lokal begrenzte Bereich, in dem eine Verstärkung der Anregung der Kernspins erfolgt, innerhalb des Stents (1) liegt.

4. Verfahren nach Anspruch 1, **dadurch gekennzeichnet**, daß bei Einstrahlung der hochfrequenten Strahlung der Schwingkreis verstimmt oder die Kapazität kurzgeschlossen wird, so daß keine verstärkte Anregung der Kernspins in dem lokal begrenzten Bereich erfolgt, bei Messung der Signalantwort des lokal begrenzten Bereichs die Verstimmung des Schwinkreises bzw. der Kurzschluß der Kapazität jedoch aufgehoben wird, was zu einer Veränderung der Signalantwort führt.

5. Verfahren nach mindestens einem der vorangehenden Ansprüche, **dadurch gekennzeichnet,** daß der Schwingkreis nach Einbringen des Stents in das Untersuchungsobjekt durch Auffalten des Stents auf die Resonanzfrequenz eingestellt wird.

6. Verfahren nach mindestens einem der vorangehenden Ansprüche, **dadurch gekennzeichnet,** daß zur resonanten Abstimmung des Schwingkreises die Induktivität und/oder die Kapazität eingestellt werden.

7. Verfahren nach mindestens einem der vorangehenden Ansprüche, **dadurch gekennzeichnet,** daß mindestens zwei am Stent ausgebildete oder angeordnete Schwingkreise verwendet werden, wobei die Spulen der jeweiligen Induktivitäten unterschiedlich, insbesondere senkrecht zueinander ausgerichtet oder hintereinander angeordnet sind.

8. Stent mit einem auffaltbaren Gerüst
**gekennzeichnet durch**
mindestens einen Schwingkreis (4, 4''') mit einer Induktivität (2, 2', 2'', 2''') und einer Kapazität (3, 3', 3'''), dessen Resonanzfrequenz im wesentlichen gleich der Resonanzfrequenz der eingestrahlten hochfrequenten Strahlung eines MR-Bildgebungssystems ist, wobei das auffaltbare Gerüst des Stents die Induktivität (2, 2'', 2''') ausbildet oder die Induktivität (2', 2'', 2''') in das Gerüst derart integriert ist, daß sie sich beim Entfalten des Stents zusammen mit dem Gerüst auffaltet.

9. Stent nach Anspruch 8, **dadurch gekennzeichnet,** daß das Gerüst aus einem Material besteht, das mindestens eine Schicht (82) mit guter Leitfähigkeit aufweist, die die Induktivität ausbildet.

10. Stent nach Anspruch 9, **dadurch gekennzeichnet,** daß das Stentmaterial mindestens zwei Schichten (81, 82) aufweist, mindestens eine Schicht (82) mit guter Leitfähigkeit und eine Schicht (81) mit schlechter Leitfähigkeit, die das Material für die eigentliche Stentfunktion bildet.

11. Stent nach Anspruch 9 oder 10, **dadurch gekennzeichnet,** das die Schicht (82) guter Leitfähigkeit an geeigneten Stellen (91) durchtrennt ist, so daß verschiedene gegeneinander isolierte Bereiche des Gerüsts derart vorliegen, daß eine Induktivität gebildet wird.

12. Stent nach Anspruch 11, **dadurch gekennzeichnet,** daß das Gerüst eine wabenförmige Struktur (101) aufweist, deren leitfähige Schicht zur Ausbildung einer Spulenanordnung regelmäßig oberhalb und unterhalb von Kreuzungspunkten der wabenförmigen Struktur (101) durchtrennt ist.

13. Stent nach mindestens einem der Ansprüche 8 bis 12, **dadurch gekennzeichnet,** daß das Gerüst (2, 2', 2'') des Stents in Form einer Helix, einer Doppel- oder einer Mehrfachhelix ausgebildet ist.

14. Stent nach Anspruch 8, **dadurch gekennzeichnet,** daß die Induktivität (2') des Schwingkreises durch eine gesonderte Spule (5) gebildet wird, die sich beim Entfalten des Stents zusammen mit dem Gerüst auffaltet.

15. Stent nach mindestens einem der Ansprüche 8 bis 14, **dadurch gekennzeichnet,** daß die Kapazität des Stents zumindest teilweise aus dem Stent-Material gebildet ist, insbesondere durch zwei Schlaufen (21, 22) der Induktivität, wobei zwischen den Schlaufen ein Dielektrikum (6) angeordnet ist.

16. Stent nach mindestens einem der Ansprüche 8 bis 14, **dadurch gekennzeichnet,** daß die Kapazität des Stents durch einen gesondert vorgesehenen Kondensator, insbesondere Plattenkondensator oder Zylinderkondensator gebildet ist.

17. Stent nach mindestens einem der Ansprüche 8 bis 16, **dadurch gekennzeichnet,** daß der Stent Mittel (13) zur Verstimmung mindestens eines Schwingkreises bei Einstrahlung der hochfrequenten Strahlung aufweist.

18. Stent nach Anspruch 17, **dadurch gekennzeichnet,** daß die Mittel zur Verstimmung des mindestens eines Schwingkreises derart ausgebildet sind, daß sie einen Kondensator (13) bei Einstrahlung der hochfrequenten Strahlung parallel zur Kapazität (3''') des Schwingkreises schalten.

19. Stent nach Anspruch 17, **dadurch gekennzeichnet**, daß die Mittel zur Verstimmung des mindestens einen Schwingkreises derart ausgebildet sind, daß sie eine Spule (14) bei Einstrahlung der hochfrequenten Strahlung parallel zur Induktivität (2''') des Schwingkreises schalten.

20. Stent nach mindestens einem der Ansprüche 8 bis 16, **dadurch gekennzeichnet,** daß der Stent Mittel (12) zum Kurzschließen der Kapazität (3''') bei Einstrahlung der hochfrequenten Strahlung aufweist.

21. Stent nach Anspruch 20, **dadurch gekennzeichnet,** daß die Mittel zum Kurzschließen der Kapazität zwei gekreuzte Dioden (12) aufweisen, die parallel zur Kapazität geschaltet (3''') sind.

22. Stent nach mindestens einem der Ansprüche 8 bis 21, **dadurch gekennzeichnet,** daß ein Schalter (10) vorgesehen ist, durch den der mindestens eine Schwingkreis aktivierbar bzw. deaktivierbar ist.

23. Stent nach mindestens einem der Ansprüche 8 bis 22, **dadurch gekennzeichnet,** daß die Induktivität (2) und/ oder die Kapazität (3) des Schwingkreises zur Abstimmung auf die Resonanzfrequenz des MR-Systems verstellbar sind.

24. Stent nach mindestens einem der Ansprüche 8 bis 23, **dadurch gekennzeichnet**, daß der Stent derart ausgebildet ist, daß bei einer Änderung der Geometrie des Stents bei dessen Applikation das Produkt aus Induktivität und Kapazität des Schwingkreises im wesentlichen konstant bleibt, insbesondere eine Erhöhung des Induktivität mit einer Verringerung der Kapazität einhergeht.

25. Stent nach mindestens einem der Ansprüche 8 bis 24, **dadurch gekennzeichnet,** daß die Güte des Schwingkreises (4) relativ gering ist.

26. Stent nach mindestens einem der Ansprüche 8 bis 25, **dadurch gekennzeichnet,** daß der Schwingkreis (4) mehrere parallel oder seriell geschaltete Induktivitäten (2a, 2n) und/oder Kapazitäten (3a, 3n) aufweist.

27. Stent nach mindestens einem der Ansprüche 8 bis 26, **dadurch gekennzeichnet,** daß der Stent mehrere Schwingkreise (2'', 7; 4a, 4b) mit mehreren Induktivitäten aufweist, die unterschiedlich, insbesondere senkrecht zueinander ausgerichtet oder hintereinander angeordnet sind .

## Claims

1. MR imaging method for representing and determining the position of a stent with an unfoldable skeleton introduced into an object to be investigated, wherein
a) the object to be investigated is placed in an external magnetic field,
b) by irradiating high-frequency radiation of a certain resonance frequency, transitions between the spin energy levels of the atomic nuclei of the object being investigated are excited, and
c) the MR signals thereby produced are detected, evaluated and represented in spatial resolution,
**characterised in that**
a changed signal response is produced in a locally restricted area in or around the stent, in that the skeleton of the stent forms at least one resonance circuit with an inductance and a capacitance or such resonance circuit is integrated in the skeleton, such that the inductance unfolds together with the skeleton during unfolding of the stent, whereby the resonance frequency of the resonance circuit is substantially equal to the resonance frequency of the radiated high-frequency radiation, and in that the area with the changed signal response is represented in spatial resolution.

2. Method according to claim 1 **characterised in that** during irradiation with the high frequency radiation the resonance circuit is excited and an amplified excitation of the nuclear spin of the investigation object thereby takes place in the locally restricted area.

3. Method according to claim 2 **characterised in that** the locally restricted area in which an amplification of the excitation of the nuclear spin takes place lies within the stent (1).

4. Method according to claim 1 **characterised in that** during irradiation with the high frequency radiation the resonance circuit is detuned or the capacitance shortcircuited so that no amplified excitation of the nuclear spin takes place in the locally restricted area, but when measuring the signal response of the locally restricted area the detuning of the resonance circuit or the short-circuiting of the capacitance is canceled which leads to a change in the signal response.

5. Method according to at least one of the preceding claims **characterised in that** the resonance circuit after introducing the stent into the investigation object is adjusted to the resonance frequency by unfolding the stent.

6. Method according to at least one of the preceding claims **characterised in that** the inductance and/or the capacitance are adjusted for the resonant tuning of the resonance circuit.

7. Method according to at least one of the preceding claims **characterised in that** at least two resonance circuits mounted or formed on the stent are used whereby the coils of each inductance are aligned differently, more particularly at right angles to each other or are arranged in succession with each other.

8. Stent with an unfoldable skeleton,
**characterised by**
at least one resonance circuit (4, 4''') with an inductance (2, 2', 2'', 2''') and a capacitance (3, 3', 3''') whose resonance frequency is substantially equal to the resonance frequency of the radiated high-frequency radiation of an MR imaging system, wherein the unfoldable skeleton of the stent forms the inductance (2, 2'', 2''') or the inductance (2', 2'', 2''') is integrated in the skeleton so that it unfolds together with the skeleton during unfolding of the stent.

9. Stent according to claim 8 **characterised in that** the skeleton consists of a material which has at least one layer (82) with good conductivity which forms the inductance.

10. Stent according to claim 9 **characterised in that** the stent material has at least two layers (81, 82), at least one layer (82) with good conductivity and one layer (81) with poor conductivity which forms the material for the actual stent function.

11. Stent according to claim 9 or 10 **characterised in that** the layer (82) of good conductivity is separated through at suitable places (1) so that different mutually insulated areas of the skeleton arise so that an inductance is formed.

12. Stent according to claim 11 **characterised in that** the skeleton has a honeycomb structure (101) whose conductive layer is separated through regularly above and below intersection points of the honeycomb structure (101) to form a coil assembly.

13. Stent according to at least one of claims 8 to 12 **characterised in that** the skeleton (2, 2', 2'') of the stent is formed in the shape of a helix, a double or a multi helix.

14. Stent according to claim 8 **characterised in that** the inductance (2') of the resonance circuit is formed by a separate coil (5) which folds open together with the skeleton during unfolding of the stent.

15. Stent according to at least one of claims 8 to 14 **characterised in that** the capacitance of the stent is formed at least in part of the stent material, more particularly through two loops (21, 22) of the inductance wherein a dielectric (6) is mounted between the loops.

16. Stent according to at least one of claims 8 to 14 **characterised in that** the capacitance of the stent is formed by a separately provided capacitor, more particularly a plate capacitor or cylinder capacitor.

17. Stent according to at least one of claims 8 to 16 **characterised in that** the stent has means (13) for detuning at least one resonance circuit during irradiation with the high-frequency radiation.

18. Stent according to claim 17 **characterised in that** the means for detuning the at least one resonance circuit are formed so that they switch a capacitor (13) during irradiation with the high frequency radiation parallel to the capacitance (3''') of the resonance circuit.

19. Stent according to claim 17 **characterised in that** the means for detuning the at least one resonance circuit are formed so that they switch a coil (1) during irradiation with the high-frequency radiation parallel to the inductance (2''').

20. Stent according to at least one of claims 8 to 16 **characterised in that** the stent has means (12) for short-circuiting the capacitance (3''') during irradiation with the high frequency radiation.

21. Stent according to claim 20 **characterised in that** the means for short-circuiting the capacitance have two crossed diodes (12) which are switched parallel to the capacitance (3"').

22. Stent according to at least one of claims 8 to 21 **characterised in that** a switch (10) is provided through which the at least one resonance circuit can be activated or deactivated.

23. Stent according to at least one of claims 8 to 22 **characterised in that** the inductance (2) and/or the capacitance (3) of the resonance circuit are adjustable to tune to the resonance frequency of the MR system.

24. Stent according to at least one of claims 8 to 23 **characterised in that** the stent is designed so that with a change in the geometry of the stent during its application the product of the inductance and capacitance of the resonance circuit remains substantially constant, more particularly an increase in the inductance is accompanied by a reduction in the capacitance.

25. Stent according to at least one of claims 8 to 24 **characterised in that** the quality of the resonance circuit (4) is relatively low.

26. Stent according to at least one of claims 8 to 25 **characterised in that** the resonance circuit (4) has several inductances (2a, 2n) and/or capacitances (3a, 3n) connected in series or in parallel.

27. Stent according to at least one of claims 8 to 26 **characterised in that** the stent has several resonance circuits (2", 7; 4a, 4b) with several inductances which are aligned differently, more particularly at right angles to each other or are arranged in succession with each other.

## Revendications

1. Procédé d'imagerie à résonance magnétique pour représenter et déterminer la position d'un écarteur introduit dans un objet à examiner et possédant une structure déployable, selon lequel
a) on dispose l'objet à examiner dans un champ magnétique extérieur,
b) on excite les transitions entre des niveaux d'énergie de spin des noyaux atomiques de l'objet à examiner en effectuant une irradiation avec un rayonnement à haute fréquence, ayant une fréquence de résonance déterminée, et
c) on détecte, on exploite et on représente selon une résolution locale des signaux obtenus de résonance magnétique, en tant que réponse de signal,
caractérisé en ce
qu'une réponse de signal modifiée est produite dans une zone limitée localement dans ou autour de l'écarteur, par le fait que la structure de l'écarteur forme ou intègre en elle un circuit oscillant avec une inductance ou une capacité de telle sorte que l'inductance se déploie lors du déploiement de l'écarteur conjointement avec la structure, la fréquence de résonance du circuit oscillant étant sensiblement égale à la fréquence de résonance du rayonnement à haute fréquence projeté, et la zone est représentée, avec une résolution locale, avec une réponse de signal modifiée.

2. Procédé selon la revendication 1, caractérisé en ce que lors de la projection du rayonnement à haute fréquence, le circuit oscillant est excité et il se produit de ce fait, dans la zone limitée localement, une excitation amplifiée des spins nucléaires de l'objet à examiner.

3. Procédé selon la revendication 2, caractérisé en ce que la zone limitée localement, dans laquelle s'effectue une amplification de l'excitation des spins nucléaires, est située à l'intérieur de l'écarteur (1).

4. Procédé selon la revendication 1, caractérisé en ce que lors de la projection du rayonnement à haute fréquence, le circuit oscillant est désaccordé ou la capacité est court-circuitée de sorte qu'il ne se produit aucune excitation amplifiée des spins nucléaires dans la zone limitée localement, mais que lors de la mesure de la réponse de signal de la zone limitée localement, le désaccord du circuit oscillant ou le court-circuit de la capacité, est supprimé, ce qui conduit à une modification de la réponse de signal.

5. Procédé selon au moins l'une des revendications précédentes, caractérisé en ce qu'après l'introduction de l'écarteur dans l'objet à examiner, le circuit oscillant est réglé par déploiement de l'écarteur, sur la fréquence de résonance.

6. Procédé selon au moins l'une des revendications précédentes, caractérisé en ce que pour le réglage d'accord à la résonance du circuit oscillant, on règle l'inductance et/ou la capacité.

7. Procédé selon au moins l'une des revendications précédentes, caractérisé en ce qu'on utilise au moins deux circuits oscillants formés ou disposés sur l'écarteur, les bobines des inductances respectives étant disposées différemment, notamment en étant orientées perpendiculairement l'une à l'autre ou en étant disposées l'une derrière l'autre.

8. Ecarteur comportant une structure repliable, caractérisé par
au moins un circuit oscillant (4, 4"') comportant une inductance (2, 2", 2"') et d'une capacité (3, 3', 3"'), dont la fréquence de résonance est sensiblement égale à la fréquence de résonance du rayonnement à haute fréquence projeté d'un système d'imagerie à résonance magnétique, la structure repliable de l'écarteur formant l'inductance (2, 2", 2"') ou intégrant l'inductance (2', 2", 2"') dans la structure de telle sorte qu'il se déploie conjointement avec la structure lors du déploiement de l'écarteur.

9. Ecarteur selon la revendication 8, caractérisé en ce que la structure est formée d'un matériau, qui comporte au moins une couche (82) possédant une bonne conductivité et qui forme l'inductance.

10. Ecarteur selon la revendication 9,
caractérisé en ce que le matériau de l'écarteur comporte au moins deux couches (81, 82), au moins une couche (82) possédant une bonne conductivité et une couche (81) possédant une mauvaise conductivité, qui forme le matériau pour la fonction proprement dite de l'écarteur.

11. Ecarteur selon la revendication 9 ou 10,
caractérisé en ce que la couche (82) possédant une bonne conductivité est sectionnée en des emplacements appropriés (91), de façon à présenter différentes zones de la structure isolées les unes des autres afin de former une inductance.

12. Ecarteur selon la revendication 11,
caractérisé en ce que la structure possède une structure en nid d'abeilles (101), dont la couche conductrice est sélectionnée, pour la formation d'un dispositif de bobine, régulièrement au-dessus et au-dessous de points de croisement de la structure en nid d'abeilles (101).

13. Ecarteur selon au moins selon au moins l'une des revendications 8 à 12, caractérisé en ce que la structure (2, 2', 2") de l'écarteur est agencée sous la forme d'une hélice, d'une hélice double ou d'une hélice multiple.

14. Ecarteur selon la revendication 8,
caractérisé en ce que l'inductance (2') du circuit oscillant est formée par une bobine séparée (5), qui se déploie conjointement avec la structure lors du déploiement de l'écarteur.

15. Ecarteur selon au moins l'une des revendications 8 à 14, caractérisé en ce que la capacité de l'écarteur est formée au moins en partie par le matériau de l'écarteur, notamment par deux boucles (21, 22) de l'inductance, un diélectrique (6) étant disposé entre les boucles.

16. Ecarteur selon au moins l'une des revendications 8 à 14, caractérisé en ce que la capacité de l'écarteur est formée par un condensateur prévu séparément, notamment un condensateur à plaques ou un condensateur cylindrique.

17. Ecarteur selon au moins l'une des revendications 8 à 16, caractérisé en ce que l'écarteur comporte des moyens (13) pour désaccorder au moins un circuit oscillant lors de la projection du rayonnement à haute fréquence.

18. Ecarteur selon la revendication 17,
caractérisé en ce que les moyens pour désaccorder le au moins un circuit oscillant sont agencés de telle sorte que lors de la projection du rayonnement à haute fréquence ils branchent un condensateur (13) en parallèle avec la capacité (3"') du circuit oscillant.

19. Ecarteur selon la revendication 17,
caractérisé en ce que les moyens pour désaccorder le au moins un circuit oscillant sont agencés de telle sorte lors de la projection du rayonnement à haute fréquence qu'ils branchent une bobine (14) en parallèle avec l'inductance (2"') du circuit oscillant.

20. Ecarteur selon au moins l'une des revendications 8 à 16, caractérisé en ce que l'écarteur comporte des moyens (12) pour court-circuiter la capacité (3"') lors de l'irradiation avec le rayonnement à haute fréquence.

21. Ecarteur selon la revendication 20,
caractérisé en ce que les moyens pour court-circuiter la capacité comportent deux diodes croisées (12), qui sont branchées en parallèle avec la capacité (3"').

22. Ecarteur selon au moins l'une des revendications 8 à 21, caractérisé en ce qu'il est prévu un interrupteur (10) au moyen duquel le au moins un circuit oscillant peut être activé ou désactivé.

23. Ecarteur selon au moins l'une des revendications 8 à 22, caractérisé en ce que l'inductance (2) et/ou la capacité (3) du circuit oscillant peuvent être déplacées pour réaliser le réglage d'accord sur la fréquence de résonance du système à résonance magnétique.

24. Ecarteur selon au moins l'une des revendications 8 à 23, caractérisé en ce que l'écarteur est agencé de telle sorte que, dans le cas d'une modification de la géométrie de l'écarteur, lors de son application, le produit de l'inductance par la capacité du circuit oscillant reste essentiellement constant, et notamment il se produit un accroissement de l'inductance avec une réduction de la capacité.

25. Ecarteur selon au moins l'une des revendications 8 à 24, caractérisé en ce que la qualité du circuit oscillant (4) est relativement faible.

26. Ecarteur selon au moins l'une des revendications 8 à 25, caractérisé en ce que le circuit oscillant (4) comporte plusieurs inductances (2a, 2n) et/ou capacité (3a, 3n) branchées en parallèle ou en série.

27. Ecarteur selon au moins l'une des revendications 8 à 26, caractérisé en ce que l'écarteur comporte plusieurs circuits oscillants (2", 7; 4a, 4b) comportant plusieurs inductances, qui sont disposées différemment et notamment sont orientées perpendiculairement entre elles ou sont disposées les unes derrière les autres.
